(19) Europäisches Patentamt
European Patent Office
Office européen des brevets

(11) **EP 1 455 931 B1**

(12) **EUROPÄISCHE PATENTSCHRIFT**

(45) Veröffentlichungstag und Bekanntmachung des Hinweises auf die Patenterteilung:
**08.08.2007 Patentblatt 2007/32**

(21) Anmeldenummer: **02791781.4**

(22) Anmeldetag: **05.12.2002**

(51) Int Cl.:
*B01J 19/32* (2006.01)  *B01J 8/00* (2006.01)
*B01J 23/10* (2006.01)  *B01J 8/02* (2006.01)
*C07C 45/28* (2006.01)  *B01D 3/00* (2006.01)

(86) Internationale Anmeldenummer:
**PCT/EP2002/013796**

(87) Internationale Veröffentlichungsnummer:
**WO 2003/047747 (12.06.2003 Gazette 2003/24)**

(54) **VORRICHTUNG UND VERFAHREN ZUR DURCHFÜHRUNG VON HETEROGEN KATALYSIERTEN REAKTIVDESTILLATIONEN, INSBESONDERE ZUR HERSTELLUNG VON PSEUDOIONON**

DEVICE AND METHOD FOR CARRYING OUT HETEROGENEOUSLY-CATALYSED REACTIVE DISTILLATIONS IN PARTICULAR FOR THE PRODUCTION OF PSEUDOIONONE

DISPOSITIF ET PROCEDE DE MISE EN OEUVRE DE DISTILLATIONS REACTIVES A CATALYSE HETEROGENE NOTAMMENT DESTINEES A LA FABRICATION DE PSEUDOIONONE

(84) Benannte Vertragsstaaten:
**AT BE BG CH CY CZ DE DK EE ES FI FR GB GR IE IT LI LU MC NL PT SE SI SK TR**

(30) Priorität: **06.12.2001 DE 10159821**

(43) Veröffentlichungstag der Anmeldung:
**15.09.2004 Patentblatt 2004/38**

(73) Patentinhaber: **BASF Aktiengesellschaft**
**67056 Ludwigshafen (DE)**

(72) Erfinder:
• **KAIBEL, Gerd**
  **68623 Lampertheim (DE)**
• **MILLER, Christian**
  **67152 Ruppertsberg (DE)**
• **DOBLER, Walter**
  **68723 Schwetzingen (DE)**
• **DIRNSTEINER, Thomas**
  **55116 Mainz (DE)**
• **SIGL, Marcus**
  **68167 Mannheim (DE)**
• **JANSEN, Helmut**
  **41542 Dormagen (DE)**
• **KAIBEL, Björn**
  **40723 Hilden (DE)**

(74) Vertreter: **Isenbruck, Günter**
**Isenbruck, Bösl, Hörschler, Wichmann, Huhn**
**Patentanwälte**
**Theodor-Heuss-Anlage 12**
**68165 Mannheim (DE)**

(56) Entgegenhaltungen:
WO-A-95/01834    WO-A-99/48604
DE-A- 4 201 456    US-B1- 6 299 845

**Beschreibung**

Die Erfindung betrifft eine Kolonne zur Durchführung von Reaktivdestillationen in Gegenwart eines heterogenen teilchenförmigen Katalysators, ein Verfahren zur Reaktivdestillation sowie eine Verwendung.

Im Stand der Technik sind verschiedene Realisierungsmöglichkeiten zur Durchführung von heterogen katalysierten Reaktivdestillationen, das heißt von heterogen katalysierten Reaktionen und gleichzeitig destillativen Auftrennungen in derselben Kolonne bekannt: eine Möglichkeit besteht darin, Packungen in aus der Destillationstechnik bekannter Bauform mit der aktiven Katalysatormasse zu beschichten, wie zum Beispiel bei der KATAPAK-M Packung der Fa. Sulzer AG, CH8404 Winterthur. Nachteilig ist hierbei, dass eine eigene Katalysatorentwicklung erforderlich ist, um aktive Katalysatormassen bereitzustellen, die auf Destillationspackungen aufgebracht werden können, dass die Haftfestigkeit der aktiven Katalysatormassen auf den Packungen häufig begrenzt ist und dass relativ begrenzte Mengen an aktiver Katalysatormasse aufgebracht werden können.

Vorteilhafter sind daher Kolonnen zur Reaktivdestillation, die Packungen in Verbindung mit teilchenförmigen Katalysatoren aufweisen. Hierzu ist es bekannt, die Katalysatorteilchen in Taschen aus Drahtgewebe einzubringen, die entweder direkt als Destillationseinbauten dienen, wie die Bauform KATAPAK-S der Fa. Sulzer AG, CH8404 Winterthur, oder die als ebene Taschen ausgebildet sind, die zwischen die einzelnen Schichten der Destillationspackungen eingelegt werden, wie die Bauform Multipak der Fa. Montz GmbH, D-40723 Hilden. Auch diese Bauformen sind jedoch hinsichtlich der Katalysatormenge, die untergebracht werden kann, begrenzt und darüber hinaus störungsanfällig im Betrieb, da vorgegebene Flüssigkeitsberieselungsdichten genau eingehalten werden müssen, was in der Praxis schwierig ist.

Von ähnlicher Bauform sind die sogenannten Bales der Fa. CDTech, Houston, USA; die Taschenstrukturen sind jedoch wesentlich gröber und daher sind die erreichbaren Trennleistungen niedriger. Sie sind beispielsweise in EP-A- 0 466 954 beschrieben.

Allen Bauformen mit in Taschen eingebrachten Katalysatorteilchen ist gemeinsam, dass das Einfüllen und Entfernen der Katalysatorteilchen umständlich und zeitaufwändig ist.

Demgegenüber sind Bauformen weniger aufwändig, bei denen der Katalysator auf Kolonnenböden aufgeschüttet wird und dort in der Flüssigkeit suspendiert ist oder in den Ablaufschächten der Kolonnenböden untergebracht ist. Diese Ausführungsformen eignen sich jedoch nur für sehr abriebfeste Katalysatoren, was in der Praxis selten zutrifft.

Aufgabe der Erfindung war es, den Einsatz herkömmlicher teilchenförmiger Katalysatoren in Reaktivdestillationskolonnen zu ermöglichen und dabei ein einfaches Einfüllen des frischen Katalysators sowie Austragen des verbrauchten Katalysators zu gewährleisten, die mechanische Beanspruchung des teilchenförmigen Katalysators durch Vermeidung von Sprudelschichten und durch ein zu großes Eigengewicht bei großen Schütthöhen zu verringern und darüber hinaus die Gas- und Flüssigkeitsströmung über den Kolonnenquerschnitt zu vergleichmäßigen.

Die Lösung geht aus von einer Kolonne zur Durchführung von Reaktivdestillationen in Gegenwart eines heterogenen teilchenförmigen Katalysators, mit einer Packung die im Kolonneninnenraum Zwischenräume ausbildet.

Die Erfindung ist dadurch gekennzeichnet, dass der Quotient aus dem hydraulischen Durchmesser für den Gasstrom durch die Packung und dem äquivalenten Durchmesser der Katalysatorteilchen im Bereich von 2 bis 20, bevorzugt im Bereich von 5 bis 10 liegt, dergestalt, dass die Katalysatorteilchen lose unter Einwirkung der Schwerkraft in die Zwischenräume eingebracht, verteilt und ausgetragen werden.

Es wurde somit gefunden, dass es möglich ist, eine mit Packungen bestückte Kolonne unmittelbar mit Katalysatorteilchen zu befüllen, ohne dass hierzu eine Ausbildung von zusätzlichen Aufnahmeräumen, beispielsweise von Taschen, erforderlich wäre.

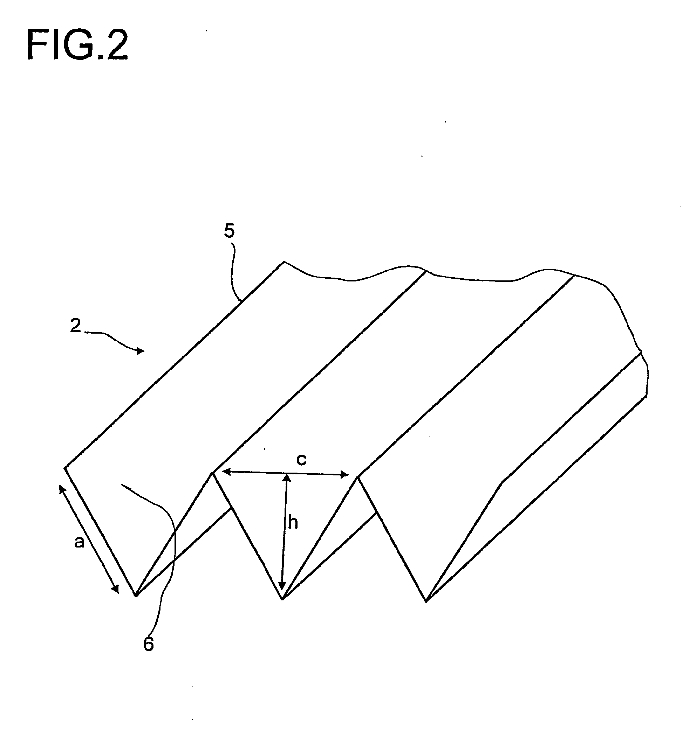

Der hydraulische Durchmesser wird in bekannter Weise als das Verhältnis zwischen dem Vierfachen der durchströmten Fläche und dem Umfang derselben definiert. Die konkrete Berechnung desselben für eine Packung mit geradlinigen Knicken ist in der Figurenbeschreibung, in Verbindung mit Figur 2 erläutert.

Die Bestimmung des hydraulischen Durchmessers von Füllkörpern erfolgt über die Porosität der Schüttung $\psi$, d. h. Leervolumen der Schüttung/Gesamtvolumen und dem äquivalenten Durchmesser der Füllkörper,

$$d_{hydraulisch} = \frac{d_p \times \psi}{1 - \psi},$$

wobei $d_{hydraulisch}$ = hydraulischer Durchmesser, $d_p$ = Durchmesser der Füllkörper und $\psi$ = Porosität. Der äquivalente Durchmesser der Füllkörper wird durch das Verhältnis zwischen dem sechsfachen Volumen und der Oberfläche des Füllkörpers definiert (vgl. VDI Wärmeatlas, 5. Auflage, 1988, Lk 1).

Der äquivalente Durchmesser von Teilchen, vorliegend Katalysatorteilchen wird durch das Verhältnis zwischen dem sechsfachen Volumen und der Oberfläche des Teilchens definiert (vgl. hierzu VDI Wärmeatlas, 5. Auflage, 1988, Lk 1.

Indem ein Quotient aus dem hydraulischen Durchmesser für den Gasstrom durch die Packung und dem äquivalenten Durchmesser der Katalysatorteilchen im oben definierten Bereich eingehalten wird, wird erfindungsgemäß gewährleistet, dass die Katalysatorteilchen lose unter Einwirkung der Schwerkraft in die Zwischenräume der Packung eingebracht, verteilt und ausgetragen werden.

Bezüglich der einsetzbaren Packungen gibt es grundsätzlich keine Einschränkungen: es können die Kolonneneinbauten verwendet werden, die regelmäßig in der Destillationstechnik eingesetzt werden, um die Phasengrenzfläche zwischen den sich im Gegenstrom durch die Kolonne bewegenden Phasen, der gasförmigen und der flüssigen Phase, zu vergrößern. Dabei bildet die Packungen im Kolonneninnenraum Zwischenräume aus, die grundsätzlich untereinander verbunden sein müssen, um die für die destillative Trennwirkung erforderliche gegensinnige Durchströmung von gasförmiger und flüssiger Phase zu gewährleisten.

Die Erfinder haben somit erkannt, dass es prinzipiell möglich ist, in die untereinander verbundenen Zwischenräume, die die Packung Kolonneninnenraum ausbilden, Katalysatorteilchen lose unter Einwirkung der Schwerkraft in die Zwischenräume einzubringen, zu verteilen und die verbrauchten Katalysatorteilchen wieder auszutragen.

Dabei ist zu beachten, dass genügend freie Zwischenräume für den bei der Destillation entstehenden Gasstrom vorhanden sind, so dass es nicht zu einem Anstauen des im Gegenstrom zur Gasströmung fließenden Flüssigkeitsstromes kommt. Dies wird erfindungsgemäß dadurch gewährleistet, dass der Quotient aus dem hydraulischen Durchmesser für den Gasstrom durch die Packung und den äquivalenten Durchmesser der Katalysatorteilchen sehr klein, das heißt mit Werten in den oben definierten Bereichen, gewählt wird.

Die Erfindung ist nicht eingeschränkt bezüglich der Form und Größe der einsetzbaren Katalysatorteilchen; zur Verbesserung der Raumzeitausbeute von heterogen katalysierten Reaktionen sind jedoch hohe spezifische Oberflächen und somit kleine Katalysatorteilchen bevorzugt. In Schüttungen von Katalysatorteilchen nimmt bekanntermaßen der Druckverlust bei zunehmend kleineren Katalysatorteilchen zu und begrenzt im Falle einer Reaktivdestillation die Flüssigkeits- und Dampfdurchsätze auf unwirtschaftlich kleine Werte. Wegen der allgemein stark ausgeprägten Bachbildung der Flüssigkeit in Katalysatorschüttungen lassen sich für große Kolonnendurchmesser, die bei Anlagen im Produktionsmaßstab benötigt werden, nur geringe destillative Trennleistungen erzielen. Diese Nachteile verhinderten bisher den an sich wünschenswerten Einsatz von Katalysatorschüttungen als Trenneinbauten in Reaktivdestillationen. Demgegenüber sind erfindungsgemäß gerade kleine Katalysatorteilchen, die auch bezüglich der katalytischen Wirksamkeit bevorzugt sind, besonders geeignet zum kombinierten Einsatz mit einer Packung da sie sich umso einfacher einbringen und, je kleiner ihre Abmessungen im Vergleich zu den Abmessungen der Zwischenräume der Packung sind.

Die Katalysatorteilchen sind bevorzugt Vollkatalysatoren, es ist jedoch auch möglich, geträgerte Katalysatoren einzusetzen. Bezüglich der Formen der Katalysatorteilchen gibt es grundsätzlich keine Einschränkungen, häufig werden Voll- oder Hohlzylinder, Kugeln, Sättel oder waben- bzw. sternförmige Stränge, eingesetzt. Geeignete Abmessungen der Katalysatorteilchen betragen beispielsweise für vollzylindrische Katalysatorteilchen etwa 1,5 x 4 bis etwa 4 x 8 mm.

Erfindungsgemäß sind die Zwischenräume, die die Packung im Kolonneninnenraum ausbildet dergestalt, dass die Katalysatorteilchen lose unter Einwirkung der Schwerkraft in die Zwischenräume eingebracht, verteilt und ausgetragen werden.

Bevorzugt werden als Kolonneneinbauten strukturierte Packungen eingesetzt, das heißt in regelmäßiger Geometrie systematisch aufgebaute Packungen mit definierten Durchtrittsbereichen für die Gegenstromphasen. Packungen sind in der Regel aus im Wesentlichen parallel zueinander angeordneten Metallblechen, Streckmetall- oder Drahtgewebelagen aufgebaut. Packungen zeichnen sich gegenüber anderen Kolonneneinbauten durch höhere Belastbarkeit, eine bessere Trennwirkung und einen geringeren spezifischen Druckverlust aus. Packungen sind in der Regel aus im Wesentlich parallel zueinander angeordneten geknickten Metallblechen, Streckmetall- oder Gewebelagen aufgebaut, mit zumeist geradlinigen Knicken, die das Packungsblech, die Streckmetall- oder Gewebelage in Knickflächen unterteilen, und wobei der Neigungswinkel der Knickfläche zur Vertikalen üblicherweise 30 bis 45° betragt. Für die vorliegende Erfindung können Packungen mit einem Neigungswinkel der Knickfläche zur Vertikalen im Bereich von 10 bis 45°, bevorzugt von 30°, eingesetzt werden. Durch Anordnung von aufeinander folgenden Packungsblechen im gleichen Neigungswinkel zur Vertikalen, jedoch mit umgekehrten Vorzeichen, entstehen die bekannten Kreuzkanalstrukturen, wie sie beispielsweise die Packungen der Typen Mellapak, CY oder BX der Fa. Sulzer AG, CH-8404 Winterthur oder die Typen A3, BSH, B1 oder M der Fa. Montz GmbH, D-40723 Hilden aufweisen.

Für die Anwendung in der Reaktivdestillation werden bevorzugt spezielle Ausführungsformen von strukturierten Packungen eingesetzt, die eine erhöhte Gasströmung zulassen.

Eine besonders bevorzugte Ausführungsform ist dadurch gekennzeichnet, dass ein oder mehrere Packungsbleche mit hoher spezifischer Oberfläche mit einem oder mehreren Packungsblechen mit niedriger spezifischer Oberfläche alternierend angeordnet werden. Dadurch werden Zwischenräume mit jeweils unterschiedlichem hydraulischen Durchmesser gebildet. Besonders bevorzugt werden die spezifischen Oberflächen der Packungsbleche dergestalt gewählt, dass zum einen Zwischenräume ausgebildet werden für die der Quotient aus dem hydraulischen Durchmesser und dem äquivalenten Durchmesser der Katalysatorteilchen < als 1 ist und zum anderen Zwischenräume für die der Quotient aus dem hydraulischen Durchmesser und dem äquivalenten Durchmesser der Katalysatorteilchen > als 2 ist, insbesondere im oben definierten Bereich zwischen 2 und 20, insbesondere zwischen 5 und 10, liegt. In die erstgenannten Zwischenräume, mit einem Verhältnis aus hydraulischem Durchmesser und äquivalentem Durchmesser der Katalysatorteilchen < als 1, werden keine Katalysatorteilchen eingefüllt, dieselben werden erfindungsgemäß lediglich in die Zwischenräume eingefüllt, in denen der genannte Quotient > als 2 ist. Durch diese besondere Ausführungsform wird eine erhöhte Gasströmung mit niedrigen Druckverlusten gewährleistet.

Bevorzugt wird das Ausgangsmaterial für erfindungsgemäße Packungen meist zusätzlich mit Öffnungen versehen, beispielsweise mit kreisförmigen Löchern mit etwa 4 bis 6 mm Durchmesser, um die Flutgrenze der Packung anzuheben und eine höhere Kolonnenbelastung zu ermöglichen. Unter Flutgrenze einer Packung wird das Gas- bzw. Flüssigkeitsvolumen pro Zeit und Querschnittsfläche verstanden, bei dem die Rieselflüssigkeit in und oberhalb der Packung bis zum vollständigen Überfluten aufgestaut bzw. vom Gasstrom mitgerissen wird. Eine Überschreitung dieser Belastung hat eine schnelle Abnahme der Trennwirkung und einen steilen Anstieg des Druckverlustes zur Folge.

Vorteilhaft sind Packungen die horizontale Flächenanteile aufweisen. Die horizontalen Flächenanteile fangen einen Teil des Gewichts der Katalysatorteilchen auf und leiten ihn an die Kolonnenwand ab. Dadurch wird die mechanische Belastung des Katalysators reduziert.

Bevorzugt sind Packungen, die aus Packungsblechen zum vertikalen Einbau in die Kolonne gebildet sind, mit geradlinigen Knicken, die das Packungsblech in Knickflächen unterteilen, wobei der Neigungswinkel der Knickflächen zur Horizontalen im Bereich von 90 bis 45°, bevorzugt bei 60°, liegt.

Die spezifische Oberfläche von Packungen für die Destillation beträgt etwa 250 bis 750 $m^2/m^3$. Für Kolonnen zur Durchführung von heterogen katalysierten Reaktivdestillationen werden Packungen mit niedrigeren spezifischen Oberflächen, im Bereich von etwa 50 bis 250 $m^2/m^3$ bevorzugt eingesetzt.

Bei Destillationspackungen genügen Wandstärken der Metallbleche von typischerweise 0,07 bis 0,1 mm. Demgegenüber werden im Falle von heterogen katalysierten Reaktivdestillationen je nach Katalysatorgewicht und mechanischer Stabilität der Katalysatorkörner Wandstärken der Metallbleche im Bereich von 01, bis 5 mm, bevorzugt von 0,15 bis 0,3 mm verwendet.

Bevorzugt werden Packungen eingesetzt, die an ihrer Oberfläche einen verringerten Strömungswiderstand aufweisen, wobei dieser verringerte Strömungswiderstand insbesondere durch Perforationen und/oder Aufrauungen des Materials der Packung oder durch Ausbildung der Packung als Streckmetall erreicht wird. Dabei sind die Perforationen bevorzugt hinsichtlich ihrer Anzahl und Abmessungen dergestalt bemessen, dass mindestens ein Anteil von 20 %, bevorzugt ein Anteil von 40 bis 80 % des flüssigen Reaktionsgemisches diese Perforationen passiert und auf die darunter liegenden Katalysatorteilchen fließt.

In einer bevorzugten Ausführungsvariante besteht das Packungsmaterial aus Streckmetall, wobei das Packungsmaterial so ausgebildet ist, dass die als Film am Packungsmaterial ablaufende Flüssigkeit möglichst vollständig durch das Packungsmaterial nach unten ablaufen kann, wobei das Abtropfen durch Ablaufkanten unterstützt wird.

Bevorzugt sind die Perforationen in der Nähe der unteren Knickkanten der vertikal in der Kolonne angeordneten Packungsbleche vorgesehen, wie in DE-A 100 31 119 beschrieben. Dadurch wird das Fluid bevorzugt auf die Oberseite der geneigten Knickflächen geleitet und die Flüssigkeitsbelastung auf der kritischen Unterseite verringert. Hierzu werden Packungen aus Packungsblechen zum vertikalen Einbau in die Kolonne eingesetzt, mit geradlinigen Knicken, die die Packungsbleche in Knickflächen unterteilen und die eine von Knickkante zu Knickkante zu messende Breite a sowie Perforationen aufweisen und wobei ein Anteil X von mindestens 60 % der Perforationen einen Abstand b von höchstens 0,4 a zur unteren Knickkante jeder Knickfläche aufweist. Bevorzugt beträgt der Anteil der von den Perforationen eine Knickfläche eingenommenen Fläche 5 bis 40 %, insbesondere 10 bis 20 % dieser Knickfläche.

Eine weitere bevorzugte Ausführungsform ist dadurch gekennzeichnet, dass die Packung aus gewellten oder geknickten Lagen ausgebildet ist, und dass zwischen zwei gewellten oder geknickten Lagen jeweils eine ebene Zwischenlage angeordnet ist, wobei sich die ebenen Zwischenlagen nicht bis an den Rand der Packung erstrecken oder in der Randzone der Packung eine erhöhte Gasdurchlässigkeit, insbesondere Löcher, aufweisen, entsprechend der DE-A 196 01 558.

Es ist auch möglich, anstelle von ebenen Zwischenlagen weniger stark gewellte oder geknickte Lagen vorzusehen.

Als Randzone der Packung wird ein konzentrisches Volumenelement bezeichnet, das sich zwischen einer äußeren Zylinderfläche und einer inneren Zylinderfläche (die Packungen haben typischerweise zylindrische Form) erstreckt, wobei die äußere Zylinderfläche durch die äußeren Enden der gewellten oder geknickten Lagen definiert ist und wobei die innere Zylinderfläche durch die äußeren Enden der ebenen Lagen definiert ist. Dabei schneidet die parallel zu den Packungslagen orientierte und durch die Kolonnenachse verlaufende horizontale Verbindungslinie der inneren mit der äußeren Zylinderfläche einen bis zwanzig, vorzugsweise drei bis zehn durch jeweils nebeneinander angeordnete Lagen gebildete Kanäle. Bei nicht in die Randzone reichenden ebenen Lagen werden somit bis zu zwanzig Kanäle in der Randzone nebeneinander freigegeben. In die Randzone reichende zweite Lagen sind vorzugsweise auf 20 bis 90 %, besonders bevorzugt auf 40 bis 60 % ihrer Fläche, gasdurchlässig, also zum Beispiel mit Löchern versehen.

An den Stellen, an denen die durch die Bleche gebildeten Kanäle die Kolonnenwand berühren, kommt es zu Stauungen des aufsteigenden Gasstroms, weil die Kanäle von der Kolonnenwand abgeschlossen werden. Das führt zu einer deutlich schlechteren Trennleistung der Packung. Durch die Öffnung der Packungskanäle in der Wandzone kann diese Ursache einer verminderten Trennleistung auf einfache und wirksame Weise beseitigt werden. Das Gas kann in diesem Fall von den an der Kolonnenwand endenden Kanälen in andere Kanäle überwechseln, die es in entgegengesetzter Richtung weiterführen.

Gegenstand der Erfindung ist auch ein Verfahren zur Reaktivdestillation in einer Kolonne, die wie vorliegend beschrieben mit einer Packung in Kombination mit einer Schüttung aus Katalysatorteilchen bestückt ist. Bevorzugt wird die Kolonne hinsichtlich ihrer Gas- und Flüssigkeitsbelastung dergestalt betrieben, dass maximal 50 bis 95 %, bevorzugt 70 bis 80 % der Flutbelastung erreicht wird.

Gegenstand der Erfindung ist auch die Verwendung der vorstehend beschriebenen Kolonne und des Verfahrens zur Durchführung von heterogen katalysierten Reaktivdestillationen, insbesondere von sauer oder basisch katalysierten Gleichgewichtsreaktionen, besonders bevorzugt zur Herstellung von Pseudoionon durch Aldolisierung von Citral und Aceton an einem auf Aluminiumoxid geträgerten Praseodym-Katalysator.

Die Erfindung wird im Folgenden anhand einer Zeichnung und eines Ausfihrungsbeispiels näher erläutert.

Es zeigen im Einzelnen

Figur 1     eine schematische Darstellung einer Ausführungsform einer erfindungsgemäßen Pakkung,

Figur 2     die schematische Darstellung eines Packungsbleches mit geradlinigen Knicken und

Figur 3     die schematische Darstellung eines Packungsbleches mit Perforationen und

Figur 4     die schematische Darstellung einer Ausführungsform einer erfindungsgemäßen Kolonne.

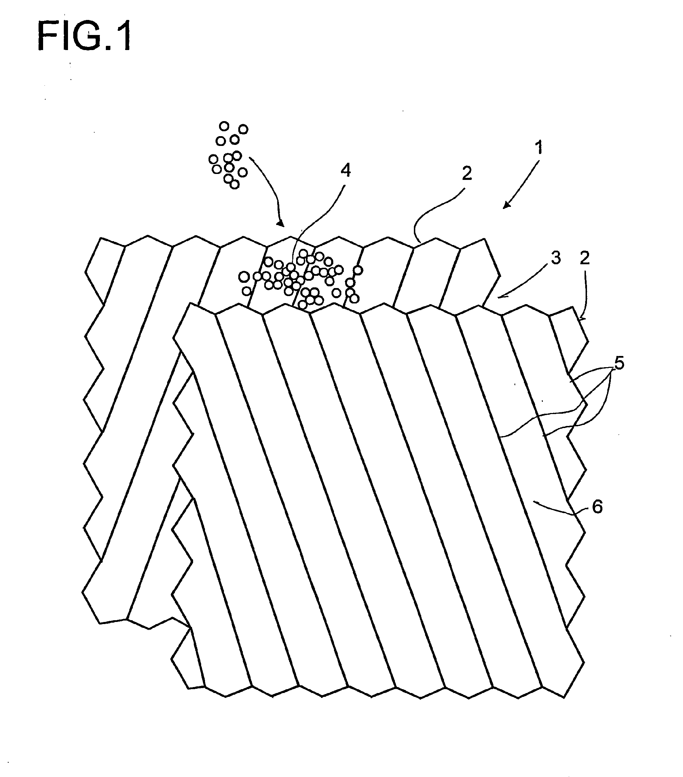

Die schematische Darstellung in Fig. 1 zeigt eine Packung 1 mit Packungsblechen 2, die geradlinige Knicke 5 unter Ausbildung von Knickflächen 6 aufweisen, wobei zwischen zwei aufeinanderfolgenden Packungsblechen 2 jeweils ein Zwischenraum 3 ausgebildet wird. In denselben werden erfindungsgemäß Katalysatorteilchen 4 eingefüllt.

Fig. 2 zeigt schematisch ein Packungsblech 2 mit geradlinigen Knicken 5, und Knickflächen 6. a stellt die Breite einer Knickfläche 6, gemessen von Knickkante 5 zu Knickkante 5, dar, c den Abstand zwischen zwei benachbarten Knickkanten 5 und h die Höhe eines Knikkers.

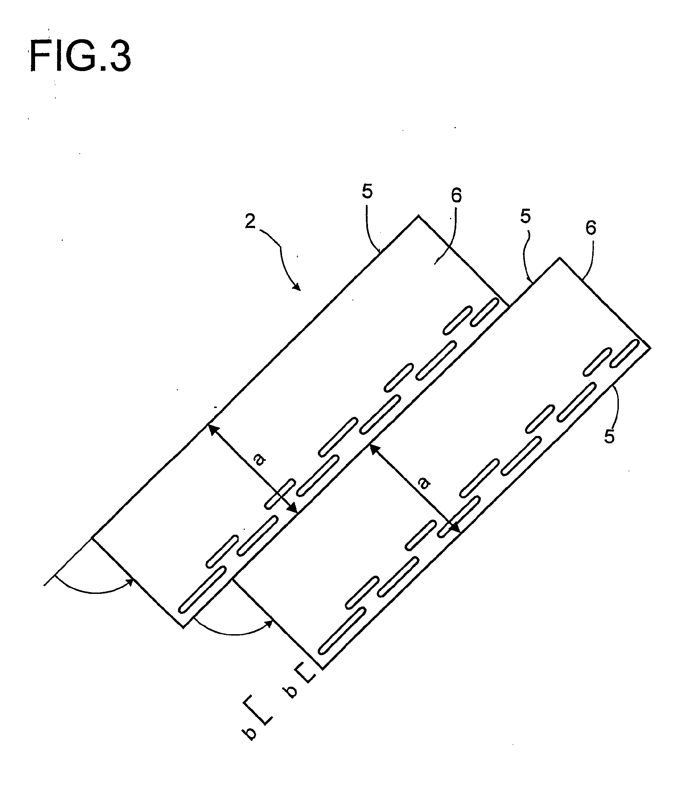

Fig. 3 zeigt schematisch eine besondere Ausführungsform eines Packungsbleches 2 mit Knickkanten 5, Knickflächen 6 und eine Breite a der Knickflächen 6 mit Perforationen, die einen Abstand b zur unteren Knickkante 5 jeder Knickfläche 6 aufweisen.

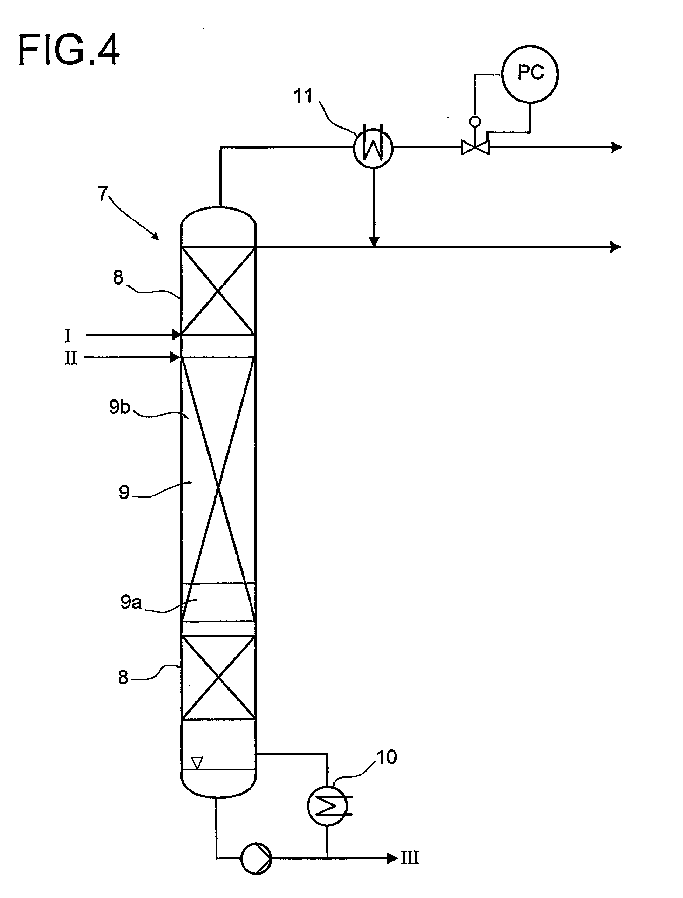

Die in der Fig. 4 schematisch dargestellte Reaktivdestillationskolonne 7 weist zwei reine Trennzonen 8, jeweils im oberen und unteren Bereich der Reaktivdestillationskolonne 7 auf, die mit strukturierten Gewebepackungen bestückt sind. Im mittleren Kolonnenbereich ist eine Reaktionszone 9 angeordnet, die einen unteren Bereich 9a mit einer Packung ohne eingebrachten Katalysatorteilchen und einen oberen Bereich 9b mit einer erfindungsgemäßen Packung, mit eingebrachten Katalysatorteilchen aufweist. Die Reaktivdestillationskolonne 7 ist mit einem Sumpfverdampfer 10 und einem Kondensator 11 am Kolonnenkopf ausgestattet. Die Edukte werden als Ströme I bzw. II im oberen Bereich der Kolonne aufgegeben, das Reaktionsgemisch wird als Sumpfstrom III abgezogen und es wird ein Kopfstrom IV am Kolonnenkopf abgezogen. Am Kolonnenkopf ist ein Druckregler PC angeordnet.

**Beispiele** (nicht erfindungsgemäß)

**Beispiel 1 Schüttversuche**

Ein Kolonnenschuss mit 0,3 m Durchmesser wurde mit zwei um 90° versetzt angeordneten Destillationspackungen des Typs B1 der Firma Montz bestückt, wobei die Höhe jeder Packung 23 cm betrug. In die Destillationspackungen wurden Katalysatorteilchen durch Schütten eingebracht. Dabei wurde das Füllvolumen und die Handhabbarkeit des Ein- und Ausbringens der Katalysatorteilchen bestimmt. Als Katalysatorteilchen wurden $\gamma$-$Al_2O_3$-und $TiO_2$-Vollzylinder eingesetzt. Die $\gamma$-$Al_2O_3$-Vollzylinder mit einem Durchmesser von 1,5 mm und einer Höhe von 1 bis 4 mm haben einen äquivalenten Partikeldurchmesser von 2 mm. Die $TiO_2$-Vollzylinder mit einem Durchmesser von 4 mm und einer Höhe von 2 bis 10 mm haben einen äquivalenten Partikeldurchmesser von 5mm.

1A) Schüttversuche mit $\gamma$-$Al_2O_3$-Vollzylindern, Durchmesser 1,5 mm. Es wurden Packungen des Typs B1 der Firma Montz mit jeweils unterschiedlichen spezifischen Oberflächen und unterschiedlichen Neigungswinkeln der Knickflächen zur Horizontalen eingesetzt.

1A$_1$) Es wurde eine Blechpackung des Typs B1-125.80 mit einer spezifischen Oberfläche von 125 $m^2/m^3$ und einem Winkel von 80° gegen die Horizontale eingesetzt. Dabei konnte 90 % des Leerrohrvolumens mit den oben genannten Katalysatorteilchen gefüllt werden. Die Pakkung hatte einen hydraulischen Durchmesser von 19 mm. Der Katalysator ließ sich sehr gut einbringen und rieselte im trockenen Zustand auch vollständig wieder heraus. Das Verhältnis des äquivalenten Durchmessers der Katalysatorteilchen zum hydraulischen Durchmesser der Packung betrug 9.

1A$_2$) Es wurde eine Packung des Typs B1-250.80 mit einer spezifischen Oberfläche von 250 m$^2$/m$^3$ und einem Winkel von 80° gegen die Horizontale mit den oben genannten Katalysatorteilchen befüllt. Hierbei konnten 80 % des Leerrohrvolumens mit Katalysatorteilchen gefüllt werden. Die Packung hat einen hydraulischen Durchmesser von 9,4 mm. Der Katalysator ließ sich sehr gut einbringen und rieselte im trockenen Zustand auch vollständig wieder heraus. Das Verhältnis des äquivalenten Durchmessers der Katalysatorteilchen zum hydraulischen Durchmesser der Packung betrug 4,7.

1A$_3$) Eingesetzt wurde eine Packung des Typs B1-250.60, das heißt mit einer spezifischen Oberfläche von 250 m$^2$/m$^3$ und einem Winkel von 60° gegen die Horizontale. 80 % des Leerrohrvolumens derselben konnten mit den oben genannten Katalysatorteilchen gefüllt werden. Die Packung hat einen hydraulischen Durchmesser von 9,4 mm. Der Katalysator ließ sich sehr gut einbringen und rieselte im trockenen Zustand auch vollständig wieder heraus. Das Verhältnis des äquivalenten Durchmesser der Katalysatorteilchen zum hydraulischen Durchmesser der Packung betrug 4,7.

1B) TiO$_2$-Vollzylinder, Durchmesser 4 mm Eingesetzt wurden die oben beschriebenen Blechpackungen des Typs B1-125.80 und B1-250.60.

1B$_1$) Eine Blechpackung des Typs B1-125.80, das heißt mit einer spezifischen Oberfläche von 125 m$^2$/m$^3$ und einem Winkel von 80° gegen die Horizontale wurde zu 80 % des Leerrohrvolumens mit den oben genannten Katalysatorteilchen gefüllt. Die Packung hat einen hydraulischen Durchmesser von 19 mm. Der Katalysator ließ sich sehr gut einbringen und rieselte im trockenen Zustand auch vollständig wieder heraus. Das Verhältnis des äquivalenten Teilchendurchmessers der Katalysatorteilchen zum hydraulischen Durchmesser der Pakkung betrug 4,5.

IB$_2$) Eine Packung des Typs B1-250.60, das heißt mit einer spezifischen Oberfläche von 250 m$^2$/m$^3$ und einem Winkel gegen die Horizontale von 60° wurde zu 50 % ihres Leerrohrvolumens mit den oben genannten Katalysatorteilchen gefüllt. Die Packung hatte einen hydraulischen Durchmesser von 9,4 mm. Der Katalysator ließ sich sehr gut einbringen und rieselte im trockenen Zustand auch vollständig wieder heraus. Das Verhältnis des äquivalenten Durchmessers der Katalysatorteilchen zum hydraulischen Durchmesser der Packung betrug 2,4.

Demgegenüber können bei handelsüblichen Katalysatorpackungen, bei denen der Katalysator in Taschen eingebracht ist, beispielsweise vom Typ Katapack der Firma Sulzer oder Multipack der Firma Montz nur 20 bis 30 % in Ausnahmefällen maximal 50 % des Leerrohrvolumens mit Katalysator gefüllt werden.

### Beispiel 2 Druckverlustmessungen

In einem Kolonnenschuss mit 0,1 m Durchmesser wurden Druckverlustmessungen mit dem Testgemisch Stickstoff/Isopropanol gemacht. Dazu wurde die Katalysatorschüttung in den Kolonnenschuss eingebracht und mit einer definierten Menge an Isopropanol beriesel (eine Tropfstelle). Im Gegenstrom hierzu wurde eine definierte Menge an Stickstoff von unten nach oben durch die Packung/Schüttung geleitet. Bei den Versuchen wurde der spezifische Druckverlust pro Packungs- bzw. Schüttungshöhe gemessen und der Flutpunkt bestimmt. Als Katalysatorteilchen wurden $\gamma$-Al$_2$O$_3$-Vollzylinder eingesetzt. Die Vollzylinder ( d = 1,5 mm, h = 1 - 4 mm) hatten einen äquivalenten Partikeldurchmesser von 2 mm. Anschließend wurde der spezifische Druckverlust und der Flutpunkt von einer in eine strukturierte Packung eingebrachten Schüttung bestimmt.

### Beispiel 2, Vergleich

Bei einer Schütthöhe von 45 cm wurde bei einem F-Faktor von 0,038 Pa^0,5 (entsprechend einem Gasstrom von 1000 1/h) und einer Berieselungsdichte von 0,178 m3/m$^2$h (entsprechend einem Flüssigkeitsstrom von 1,4 1/h) ein spezifischer Druckverlust von 3,33 mbar/m gemessen. Die Packung begann bei konstanter Flüssigkeitsbelastung von 0,178 m$^3$/m$^2$h ab einem F-Faktor von 0,0575 Pa^0,5 (entsprechend einem Gasstrom von 1500 1/h) zu fluten.

### Beispiel 2,

Schüttung eingebracht in zwei um 90° gedrehte Lagen einer strukturierten Packung des Typs BS-250.60 der Firma Montz.
Bei einer Schütthöhe von 46 cm wurde bei einem F-Faktor von 0,038 Pa^0,5 (entsprechend einem Gasstrom von 1000 1/h) und einer Berieselungsdichte von 0,178 m$^3$/m$^2$h (entsprechend einem Flüssigkeitsstrom von 1,4 1/h) ein spezifischer Druckverlust von 1,09 mbar/m gemessen. Die Packung begann bei konstanter Flüssigkeitsbealstung von 0,178 m³/m²h ab einem F-Faktor von 0,114 Pa^0,5 (entsprechend einem Gasstrom von 3000 l/h) zu fluten. Die maximale Gasbelastung konnte somit im Vergleich zur Schüttung, die nicht in eine Packung eingebracht war, um den Faktor 2 gesteigert werden.

Im Folgenden wird unter Bezugnahme auf Fig. 2 die Berechnung des hydraulischen Durchmessers für eine Packung mit geradlinigen Knicken verdeutlicht:

Das in Fig. 2 beispielhaft dargestellte Packungsblech 2 weist parallel zueinander angeordnete, geradlinige Knicke 5 auf, die das Packungsblech 2 in Knickflächen 6 unterteilen. Die Breite einer Knickfläche 6, von Knickkante 5 zu Knickkante 5 gemessen, wird mit a bezeichnet, der Abstand zwei aufeinanderfolgenden Knickkanten 5 mit c und die Höhe der Knicke mit h. Der hydraulische Durchmesser der Gasströmung für eine aus derartigen Packungsblechen aufgebaute Packung berechnet sich dann nach der Formel

$$d'_{hydraulisch,Gas} = \frac{2\,c \cdot h}{c + 2a}$$

## Beispiel 3 Herstellung von Pseudoionon durch Aldolisierung von Citral und Aceton

Die Versuchsanordnung entsprach der schematischen Darstellung in Fig. 4. Die Reaktivdestillationskolonne 7 war in den Trennzonen 8 mit jeweils einem Segment einer strukturierten Gewebepackung vom Typ A3-500 der Firma Montz, mit einer Gesamthöhe von jeweils 23 cm, gefüllt. Die Reaktionszone 9 war im unteren Bereich derselben mit einer Lage Montz-Pak Typ B1-1000 in Spezialelementhöhe 30 mm bestückt. Diese Lage diente als Katalysatorsperre, damit die Katalysatorteilchen nicht in die untere Trennzone rieseln konnten. Auf diese Lage wurden drei Lagen Montz-Pak vom Typ B1-250.60 mit einer Elementhöhe von 212 mm eingebaut, in die der Katalysator durch Schütten eingebracht wurde. Dabei wurden 3121 g Katalysator mit einer Schüttdichte von 700 kg/m³ eingeschüttet. Als Katalysator wurden Vollzylinder aus 5 % Praseodym auf $\gamma$-Al$_2$O$_3$ mit einem Teilchendurchmesser von 1,5 mm und einer Höhe von 1 bis 4 mm verwendet, die durch Tränken von $\gamma$-Al$_2$O$_3$ mit einer wässrigen Lösung von Praseodym - Nitrat und anschließender Kalzinierung hergestellt wurden. Die Kolonne war in regelmäßigen Abschnitten mit Thermoelementen sowie mit Probennahmestellen bestückt, so dass das Temperaturprofil und das Konzentrationsprofil in der Kolonne ermittelt werden konnten.

Die Reaktanden Citral und Aceton (Strom I bzw. II in Fig. 4) wurden aus auf Waagen stehenden Vorlagebehältern mit einer Pumpe massenstromgeregelt in die Reaktivdestillationskolonne dosiert.

Der Sumpfverdampfer 10, der mit Hilfe eines Thermostaten auf 124°C beheizt wurde, hatte während des Betriebs je nach Verweilzeit ein Hold-up zwischen 50 und 150 ml. Der Sumpfstrom III wurde aus dem Sumpfverdampfer 10 mit einer Pumpe standgeregelt in einen auf einer Waage stehenden Behälter gefördert.

Der Kopfstrom der Reaktivdestillationskolonne wurde in einem Kondensator 11, der mit einem Kryostaten betrieben wurde, auskondensiert. Ein Teil des Kondensats lief über einen Rücklaufteiler als Strom IV in einen auf einer Waage stehenden Vorlagebehälter, während der andere Teil als Rücklauf auf die Kolonne gegeben wurde. Die Apparatur war mit einer Druckregelung PC ausgestattet und auf einen Systemdruck von 20 bar ausgelegt. Alle ein-und austretenden Stoffströme wurden während des gesamten Versuchs mit einem Prozessleitsystem PLS kontinuierlich erfasst und registriert. Die Apparatur wurde kontinuierlich, im 24 Stunden-Betrieb, gefahren.

In die oben beschriebenen Reaktivdestillationskolonne 7 wurde kontinuierlich ein Strom 1 von 220,0 g/h, entsprechend 1,4 mol/h Citral mit einer Reinheit von 97 % sowie ein Strom II von 840,0 g/h entsprechend 14,32 mol/h auf 80°C vorgewärmtes Aceton mit einer Reinheit von 99 % aufgegeben.

## Versuchsdurchführung

Als Katalysator in der Reaktionszone 9 wurden Vollzylinder (d = 1,5 mm, h = 1 - 4 mm) aus 5 % Pr auf $\gamma$-Al$_2$O$_3$ verwendet. Es wurde ein Systemdruck von 3 bar und ein Rücklaufverhältnis von 3 kg/kg eingestellt. Die Sumpftemperatur betrug 92,5°. Als Sumpfstrom III der Kolonne wurden 735,6 g/h Rohprodukt mit 62,14 Gew.-% Aceton, 0,71 Gew.-% Wasser, 0,45 Gew.-% Mesityloxid, 0,95 Gew.-% Diacetonalkohol, 9,14 Gew.-% Citral, 24,43 Gew.-% Pseudoionon und 2,18 Gew.-% Hochsiedem gewonnen. Am Kopf der Kolonne wurden 323,2 g/h Destillat (Strom IV) bestehend aus 95,8 Gew.-% Aceton und 4,2 Gew.-% Wasser abgezogen.

Es wurde Pseudoionon mit einer Selektivität von 97,3 % bezogen auf Citral und 84,4 % bezogen auf Aceton erhalten. Die Ausbeute betrug 66,7 % bezogen auf Citral.

Bei F-Faktoren von 0,12 Pa^0,5 und Berieselungsdichten von 0,3 m³/m²h wurde ein Differenzdruck über die Kolonne von ca. 1 mbar gemessen.

Bei Verwendung einer regelosen Katalysatorschüttung ohne Packung wurde demgegenüber der doppelte Druckverlust gemessen.

Der Differenzdruck ist ein Maß für die Belastung (Gas und Flüssigkeit) der Kolonne. je nach Stoffeigenschaften und der Art der verwendeten Einbauten steigt der Differenzdruck mit zunehmender Belastung an, bis es zum Fluten kommt. Im Zustand des Flutens wird der Katalysator aufgewirbelt und es kann zu einem starken Katalysatorabrieb kommen. Dieser Zustand ist daher zu vermeiden.

Bei Verwendung einer erfindungsgemäßen Packung kann daher ein höherer Durchsatz bei gleichem Kolonnendurchmesser erreicht werden.

**Patentansprüche**

1. Kolonne zur Durchführung von Reaktivdestillationen in Gegenwart eines heterogenen teilchenförmigen Katalysators, mit einer Packung gebildet aus Packungsblechen, die im Kolonneninnenraum Zwischenräume ausbilden, **dadurch gekennzeichnet, dass** die Kolonne erste und zweite Teilbereiche aufweist, die alternierend angeordnet sind und sich durch die spezifische Oberfläche der Packungsbleche unterscheiden, dergestalt, dass in den ersten Teilbereichen der Quotient aus dem hydraulischen Durchmesser für den Gasstrom durch die Packung und dem äquivalenten Durchmesser der Katalysatorteilchen im Bereich von 2 bis 20, bevorzugt im Bereich von 5 bis 10 liegt, so dass die Katalysatorteilchen lose unter Einwirkung der Schwerkraft in die Zwischenräume eingebracht, verteilt und ausgetragen werden können und dass in den zweiten Teilbereichen der Quotient aus dem hydraulischen Durchmesser für den Gasstrom durch die Packung und dem äquivalenten Durchmesser der Katalysatorteilchen kleiner als 1 ist und dass in die zweiten Teilbereiche keine Katalysatorteilchen eingebracht werden.

2. Kolonne nach Anspruch 1, **dadurch gekennzeichnet, dass** die Packung eine strukturierte Packung ist.

3. Kolonne nach Anspruch 2, **dadurch gekennzeichnet, dass** die strukturierte Packung eine Kreuzkanalpackung ist.

4. Kolonne nach einem der Ansprüche 1 bis 3, **dadurch gekennzeichnet, dass** die Packung horizontale Flächenanteile aufweist.

5. Kolonne nach Anspruch 4, wobei die Packung aus Packungsblechen zum vertikalen Einbau in die Kolonne gebildet ist, mit geradlinigen Knicken, die das Packungsblech in Knickflächen unterteilen, **dadurch gekennzeichnet, dass** der Neigungswinkel der Knickflächen zur Horizontalen im Bereich von 90 bis 45°, bevorzugt bei 60°, liegt.

6. Kolonne nach einem der Ansprüche 1 bis 5, **dadurch gekennzeichnet, dass** die Packung an ihrer Oberfläche einen verringerten Strömungswiderstand aufweist, bevorzugt durch Perforationen und/oder Aufrauungen des Materials der Packung oder durch Ausbildung der Packung als Streckmetall.

7. Kolonne nach einem der Ansprüche 2 bis 6, **dadurch gekennzeichnet, dass** die Packung aus gewellten oder geknickten Lagen ausgebildet ist, und dass zwischen zwei gewellten oder geknickten Lagen jeweils eine ebene Zwischenlage angeordnet ist, wobei sich die ebenen Zwischenlagen nicht bis an den Rand der Packung erstrekken oder in der Randzone der Packung eine erhöhte Gasdurchlässigkeit, insbesondere Löcher, aufweisen.

8. Kolonne nach einem der Ansprüche 2 bis 7, wobei die Packung aus Packungsblechen zum vertikalen Einbau in die Kolonne gebildet ist, mit geradlinigen Knicken, die die Packungsbleche in Knickflächen unterteilen und die eine von Knickkante zu Knickkante zu messende Breite a sowie Perforationen aufweisen, **dadurch gekennzeichnet, dass** ein Anteil X von mindestens 60 % der Perforationen einen Abstand b von höchstens 0,4 a zur unteren Knickkante jeder Knickfläche aufweist.

9. Verfahren zur Reaktivdestillation in einer Kolonne nach einem der Ansprüche 1 bis 8, **dadurch gekennzeichnet, dass** die Kolonne hinsichtlich ihrer Gas- und Flüssigkeitsbelastung so betrieben wird, dass maximal 50 bis 95 %, bevorzugt 70 bis 80 % der Flutbelastung erreicht wird.

10. Verwendung einer Kolonne nach einem der Ansprüche 1 bis 8 oder eines Verfahrens nach Anspruch 9 zur Durchführung von heterogen katalysierten Reaktivdestillationen, insbesondere von sauer oder basisch katalysierten Gleichgewichtsreaktionen, besonders bevorzugt zur Herstellung von Pseudoionon durch Aldolisierung von Citral und Aceton an einem auf Aluminiumoxid geträgerten Praseodym-Katalysator.

**Claims**

1. A column for carrying out reactive distillations in the presence of a heterogeneous particulate catalyst having an ordered packing formed from ordered sheet metal packings which form intermediate spaces in the column interior, wherein the column has first and second part regions which are arranged in alternation and which differ by the specific surface area of the ordered sheet metal packings in such a manner that in the first part regions the quotient of the hydraulic diameter for the gas flow through the ordered packing and the equivalent diameter of the catalyst particles is in the range from 2 to 20, preferably in the range from 5 to 10, so that the catalyst particles can be introduced into the intermediate spaces, distributed and discharged loose under the action of gravity and in the second part regions the quotient of the hydraulic diameter for the gas stream through the ordered packing and equivalent diameter of the catalyst particles is less than 1 and no catalyst particles are introduced into the second part regions.

2. The column according to claim 1, wherein the ordered packing is a structured packing.

3. The column according to claim 2, wherein the structured packing is a cross-channel packing.

4. The column according to one of claims 1 to 3, wherein the ordered packing has horizontal surface portions.

5. The column according to claim 4, wherein the ordered packing is formed from ordered sheet metal packings for vertical installation into the column having linear corrugations which subdivide the ordered sheet metal packing into corrugated surfaces, wherein the angle of inclination of the corrugated surfaces to the horizontal is in the range from 90° to 45°, preferably 60°.

6. The column according to one of claims 1 to 5, wherein the ordered packing has a reduced resistance to flow at its surface, preferably due to perforations and/or roughness of the material of the ordered packing or by constructing the ordered packing as expanded metal.

7. The column according to one of claims 2 to 6, wherein the ordered packing is formed from rippled or corrugated layers, and between two rippled or corrugated layers in each case one flat intermediate layer is disposed, with the flat intermediate layers not extending to the edge of the ordered packing or having, in the edge zone of the ordered packing, an increased gas permeability, in particular holes.

8. The column according to one of claims 2 to 7, wherein the ordered packing is formed from ordered sheet metal packings for vertical installation into the column having linear corrugations which subdivide the ordered sheet metal packings into corrugated surfaces and which have a width a, measured from corrugated edge to corrugated edge and perforations, wherein a proportion X of at least 60% of the perforations has a distance b of at most 0.4 a to the lower corrugated edge of each corrugated surface.

9. A process for reactive distillation in a column according to one of claims 1 to 8, which comprises operating the column with respect to its gas and liquid loadings in such a manner that a maximum of from 50 to 95%, preferably from 70 to 80%, of the flooding limit loading is reached.

10. The use of a column according to one of claims 1 to 8 or of a process according to claim 9 for carrying out heterogeneously catalyzed reactive distillations, in particular acid- or base-catalyzed equilibrium reactions, particularly preferably for preparing pseudoionone by aldolizing citral and acetone in the presence of an aluminum-oxide-supported praseodymium catalyst.

**Revendications**

1. Colonne pour l'exécution de distillations réactives en présence d'un catalyseur hétérogène particulaire, comportant un garnissage formé de tôles de garnissage qui forment dans l'espace intérieur de la colonne des espaces intermédiaires, **caractérisée en ce que** la colonne présente des premières et deuxièmes zones partielles agencées en alternance et qui diffèrent par la surface spécifique des tôles de garnissage, de telle manière que, dans les premières zones partielles, le quotient du diamètre hydraulique du courant de gaz dans le garnissage et du diamètre équivalent des particules de catalyseur soit de l'ordre de 2 à 20, de préférence de l'ordre de 5 à 10, de telle sorte que les particules de catalyseur puissent être librement introduites, dispersées et déchargées sous l'action de la gravité dans les espaces intermédiaires et que, dans les deuxièmes zones partielles, le quotient du diamètre hydraulique du courant de gaz dans le garnissage et du diamètre équivalent des particules de catalyseur soit inférieur à 1 et qu'il n'y ait pas de particules de catalyseur introduites dans les deuxièmes zones partielles.

2. Colonne suivant la revendication 1, **caractérisée en ce que** le garnissage est un garnissage structuré.

3. Colonne suivant la revendication 2, **caractérisée en ce que** le garnissage structuré est un garnissage à canaux croisés.

4. Colonne suivant l'une quelconque des revendications 1 à 3, **caractérisée en ce que** le garnissage présente des parties de surface horizontales.

5. Colonne suivant la revendication 4, dans laquelle le garnissage est formé de tôles de garnissage à disposer verticalement dans la colonne, comportant des pliures rectilignes qui divisent la tôle de garnissage en surfaces angulées, **caractérisée en ce que** l'angle d'inclinaison des surfaces angulées par rapport à l'horizontale est de l'ordre de 90 à 45°C, de préférence de 60°C.

6. Colonne suivant l'une quelconque des revendications 1 à 5, **caractérisée en ce que** le garnissage présente à sa surface une moindre résistance à l'écoulement, de préférence via des perforations et/ou des rugosifications du matériau du garnissage ou par conformation du garnissage en forme de métal déployé.

7. Colonne suivant l'une quelconque des revendications 2 à 6, **caractérisée en ce que** le garnissage est formé de couches ondulées ou pliées et qu'entre deux couches ondulées ou pliées est à chaque fois placée une couche intermédiaire plane, les couches intermédiaires planes n'arrivant pas jusqu'à la périphérie du garnissage ou présentant, dans la zone périphérique du garnissage, une plus grande perméabilité aux gaz, en particulier des perforations.

8. Colonne suivant l'une quelconque des revendications 2 à 7, où le garnissage est formé de tôles de garnissage à disposer verticalement dans la colonne, comportant des pliures rectilignes qui divisent les tôles de garnissage en surfaces angulées et présentent une largeur a, mesurée d'une arête de pliure à une autre arête de pliure, ainsi que des perforations, **caractérisée en ce qu'**une fraction X d'au moins 60% des perforations présente une distance b d'un maximum de 0,4 a de l'arête de pliure inférieure de chaque surface angulée.

9. Procédé de distillation réactive dans une colonne suivant l'une quelconque des revendications 1 à 8, **caractérisé en ce que** la colonne est conduite, en ce qui concerne sa charge de gaz et de liquide, de telle sorte que l'on atteigne au maximum 50 à 95%, de préférence 70 à 80% de la charge d'irrigation.

10. Utilisation d'une colonne suivant l'une quelconque des revendications 1 à 8 ou d'un procédé suivant la revendication 9 pour l'exécution de distillations réactives à catalyse hétérogène, en particulier de réactions en équilibre à catalyse acide ou basique, plus préférablement pour la préparation de pseudoionone par aldolisation de citral et d'acétone sur un catalyseur de praséodyme sur support d'oxyde d'aluminium.

# FIG.1

# FIG.2

# FIG.3

# FIG.4

**EP 1 455 931 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0466954 A
- DE 10031119 A
- DE 19601558 A